# EUROPEAN PATENT SPECIFICATION

(11) **EP 4 157 209 B1**
(45) Date of publication and mention of the grant of the patent: **26.08.2026**
(21) Application number: 21729505.4
(22) Date of filing: 28.05.2021
(51) Int. Cl.: A61K 8/37, A61K 8/49, A61Q 19/08

(54) **COSMETIC COMPOSITION WITH ENHANCED COLOR STABILITY FOR RETINOIC ACID PRECURSOR**
KOSMETISCHE ZUSAMMENSETZUNG MIT VERBESSERTER FARBSTABILITÄT FÜR RETINOLSÄUREVORLÄUFER
COMPOSITION COSMÉTIQUE PRÉSENTANT UNE MEILLEURE STABILITÉ DE COULEUR POUR UN PRÉCURSEUR DE L'ACIDE RÉTINOÏQUE

(30) Priority: 29.05.2020 EP 20177510
(43) Date of publication of application: 05.04.2023
(73) Proprietor: Unilever IP Holdings B.V., 6708 WH Wageningen (NL); Unilever Global IP Limited, Wirral, Merseyside CH62 4ZD (GB)
(72) Inventor: BEKTO, Hasiba, Trumbull, CT Connecticut 06611 (US); HUANG, Lei, Trumbull, CT Connecticut 06611 (US); TRUONG, Hang, Nguyet, Trumbull, CT Connecticut 06611 (US)
(74) Representative: Unilever Patent Group
(86) International application number: PCT/EP2021/064332
(87) International publication number: WO 2021/239938

(56) References cited:
- EP-A1- 0 896 522
- JP-A- 2011 219 463
- JP-B2- 5 712 010

## Description

### Technical Field

The present invention is in the field of personal care compositions; in particular skin-benefit compositions for topical application to human skin. More particularly, the present invention relates to a cosmetic composition containing a retinoic acid precursor in combination with compatible oils and a functionalized heteroaromatic compound to provide for improved color stability of the composition.

### Background

Human skin is subjected to deterioration through environmental factors (wind, dry air, sun exposure, pollution, blue light), dermatological conditions, and through the normal aging process (chronoaging). Compromised skin leads to the development of facial fine lines, wrinkles, yellowing or sallowness, sagging, dehydration, hyperpigmentation, age spots and the general signs of aging. However, standard definitions of ideal skin include at present time youthful skin with even tone and smooth texture. Thus, the demand for "anti-aging" or "pre-aging" cosmetic products that reverse, treat or delay the visible signs of compromised skin has grown immensely.

Cosmetic compositions containing retinoic acid precursors, such as retinol, have become quite prominent in recent years. Retinol, also known as Vitamin A, and many of its ester derivatives and retinoid family members including, for example, retinyl propionate, can be effective in addressing the visible signs of aging skin described above, among other benefits, when applied topically. Notwithstanding the benefits of retinoids, the inclusion of retinoic acid precursors in cosmetic compositions often results in compositions having difficulties with stability.

In addition, many people often look to cosmetic products with skin-lightening benefits for desirable even skin. There exists an ever-present need in the industry for cosmetic skin-lightening agents that have enhanced efficaciousness and stability. Without intending to be bound or limited by theory, it is believed that compositions containing a functionalized heteroaromatic compound, particularly niacinamide and/or nicotinate, are useful for promoting even skin and skin hydration while reducing skin dullness and the appearance of fine lines, among other benefits. Further examples of skin-lightening agents that may be used in topical applications for the skin include resorcinols, such as hexylresorcinol, hydroquinone, arbutin and kojic acid.

However, obtaining color stable cosmetic compositions comprising retinoic acid precursors can be challenging. Color instability is of concern because consumers are sensitive to changes in color appearance of cosmetic compositions that they use, especially in cases where people may believe that color change cues degradation of chemical efficacy. The color instability of retinoic acid precursors is positively correlated in a nonlinear manner with chemical instability of retinoic acid precursors, but not in a proportional or exponential fashion. Color stability in compositions with retinoic acid precursors tends to deteriorate in a shorter time period than does chemical activity. Trace amounts of chemical degradation in retinoic acid precursors due to extrinsic factors including impurities in the formulation and interactions with other ingredients, such as niacinamide, can result in large degradations of color. Thus, cosmetic compositions desiring the inclusion of both a retinoic acid precursor and a functionalized heteroaromatic compound may face noticeable color degradation.

Thus, the present inventors have recognized a need to develop a cosmetic composition with improved color stability that includes a retinoic acid precursor and a functionalized heteroaromatic compound. It is therefore an object of the present invention to provide stable cosmetic compositions having a retinoic acid precursor and a compatible oil in combination with a functionalized heteroaromatic compound.

### Additional Information

Efforts have been disclosed for incorporating a retinoic acid precursor with a functionalized heteroaromatic compound in the presence of a compatible oil.

U.S. Patents No. 5,997,890 and 6,001,377 disclose topical compositions useful for providing immediate improvements in skin appearance containing a particulate material and an active for regulating skin conditions, preferred options which include vitamin B₃ compounds and retinoids.

U.S. Patents No. 5,933,998, 5,939,082, 6,150,403, 6,238,678 and 6,217,888 disclose topical compositions for regulating the oily and/or shiny appearance of skin, wherein a preferred embodiment contains retinoid and a vitamin B₃ compound.

U.S. Patents No. 6,224,888 and 6,528,071 relate to cosmetic compositions comprising a vitamin B₃ compound, an oil liquid emollient, a polar solvent, a solidifying agent and a coloring agent.

U.S. Patent No. 6,183,761 reports topical compositions for regulating skin appearance having a vitamin B₃ compound, a polycyclic compound, and acceptable carrier.

PCT application WO 94/09756 relates to a composition for topical application to human skin to promote the repair of photo-damaged skin comprising retinol or a derivative thereof and a selected skin-lightening agent.

PCT application WO 98/52536 reports skin care compositions with improved skin compatibility containing retinoids and a preservative component.

PCT application WO 99/47115 describes skin care compositions comprising vitamin B₃ compound and an improved carrier system.

None of the additional information above describes the particular combination of retinoic acid and a functionalized heteroaromatic compound with a compatible oil to achieve a cosmetic composition with improved color stability to be used for anti-aging or pre-aging and skin-lightening benefits as disclosed in the presently claimed invention.

### Summary

The present inventors have determined that the use of a functionalized heteroaromatic compound with a compatible oil in addition to use of a retinoic acid precursor enhances the color stability of compositions formulated with retinoic acid precursor.

Accordingly, in a first aspect, the invention relates to a cosmetic composition comprising a retinoic acid precursor, a functionalized heteroaromatic compound and a compatible oil that surprisingly enhances the color stability of the composition. The composition is disclosed in the appended claims.

The present disclosure also refers to a method of treating skin in need of treatment comprising the steps of topically applying said cosmetic composition onto skin in need of treatment.

Cosmetic compositions made according to the present invention have been unexpectedly discovered to provide good stability, color stability, anti-aging and skin-lightening benefits.

All other aspects of the present invention will become more readily apparent upon considering the detailed description and examples which follow.

For the avoidance of doubt, the term "comprising" is meant not to be limiting to any stated elements but rather to encompass non-specified elements of major or minor functional importance. Therefore, the listed steps, elements or options need not be exhaustive. Whenever the words "including" or "having" are used, these terms are meant to be equivalent to "comprising" as defined above.

The term "skin" as used herein includes the skin on the face, neck, chest, back, arms, axilla, buttocks, hands, legs and scalp. Skin benefit agent, as used herein, is meant to include a component that (a) improves a facial or body characteristic after topical application like a skin characteristic, (b) benefits the same, or (c) both (a) and (b). The personal care composition is a composition for topical application and includes a lotion, cream, gel, serum, pump or aerosol spray, balm, deodorant, antiperspirant, nail treatment, wash composition and make-up. In an especially preferred embodiment, the composition of this invention is a leave-on composition and the skin benefit agent is a retinoic acid precursor compound. The terms "retinoid" and "retinoic acid precursor," as used herein, refers to all natural and/or synthetic analogues of vitamin A or derivatives thereof having similar biological activity as vitamin A in human skin. The term "derivative(s)" as used with reference to ingredients herein refers to functional group substitutions on the compound. The term "functionalized," as used herein, means that a compound possesses at least one functional group substituent, including an acyl, hydroxy, alkoxy, carboxamide (amide), carboxyl, or carboalkoxy (ester) substituent, on the compound. "HLB" stands for the hydrophilic-lipophilic balance of a surfactant, used to measure the degree to which a surfactant is hydrophilic or lipophilic, and is widely used in the art. Remains color stable, as used herein, describes a cosmetic composition that possesses a ΔE of ten (10) or less based on L*, a* and b* color differences based on the CIELAB color space model taken on a Hunter Lab spectrophotometer. Generally, ΔE ≤10 is acceptable, ΔE between 10 to 20 is negotiable and ΔE >20 is unacceptable. For the b* value, the values represent a blue-yellow component of an object's color, wherein a negative (-) value represents movement into the blue color space while a positive (+) value represents movement into the yellow color space. A b* value of 0 represents a true neutral gray value. The term "compatible" as used herein refers to an oil possessing a Hansen total solubility parameter value within the range of 13 to 26. Hansen total solubility parameters are determined based on three Hansen parameters, namely □ₜ (defines energy from dispersion forces between molecules), □ₚ (defines energy from dipolar intermolecular forces between molecules), and □ₙ (defines energy from hydrogen bonds between molecules). □ₜ values are obtained according to the CRC Handbook of Solubility Parameters and other Cohesion Parameters, Second Edition by Allan F.M. Barton.

Except in the examples, or where otherwise explicitly indicated, all numbers in this description indicating amounts of material are to be understood as modified by the word "about." All amounts are by total weight of the composition, unless otherwise specified.

### Detailed Description

The inventive compositions contain a retinoic acid precursor, which includes retinyl esters, retinol, retinal or mixture thereof, preferably, retinal, retinyl ester or mixture thereof. Examples of retinyl esters suitable for use in the invention include but are not limited to: retinyl palmitate, retinyl formate, retinyl acetate, retinyl propionate, retinyl butyrate, retinyl valerate, retinyl isovalerate, retinyl hexanoate, retinyl heptanoate, retinyl octanoate, retinyl nonanoate, retinyl decanoate, retinyl undecandate, retinyl taurate, retinyl tridecanoate, retinyl myristate, retinyl pentadecanoate, retinyl heptadeconoate, retinyl stearate, retinyl isostearate, retinyl nonadecanoate, retinyl arachidonate, retinyl behenate, retinyl linoleate, and retinyl oleate. The preferred ester for use in the present invention is selected from retinyl palmitate, retinyl acetate, retinyl propionate and mixtures thereof. Retinyl linoleate and retinyl oleate are also preferred. In a particularly preferred embodiment, the retinoic acid precursor is retinyl proprionate. Retinoic acid precursor is employed in the inventive composition in an amount from 0.001 to 10%, preferably, in an amount from 0.01 to 1%, most preferably, in an amount from 0.01 to 0.5% by weight of the cosmetic composition.

It is often desirable to include a functionalized heteroaromatic compound in cosmetic compositions with a retinoic acid precursor based on skin benefits. Yet, it has been discovered that when a composition comprising retinoic acid precursor is further combined with a functionalized heteroaromatic compound, color instability of the retinoic acid precursor is exacerbated and manifests as a darker, yellow discoloration of the composition, a challenge which the present invention can help to overcome. Cosmetic compositions of the present invention include, in addition to the retinoic acid precursor, a functionalized heteroaromatic compound. The functionalized heteroaromatic compound is a carboxylic acid functionalized heteroaromatic compound comprising nicotinic acid, picolinic acid, nicotinate,niacinamide,picolinamide or mixture thereof, and more preferably, niacinamide, picolinamide or mixtures thereof. Amounts of the functionalized heteroaromatic compound are typically used from about 0.01 to 10%, preferably, from about 0.05 to 7%, and, more preferably, from about 0.1 to 5% by weight of the cosmetic composition.

Now it has been unexpectedly determined that use of a compatible oil in compositions with retinoic acid precursor and a functionalized heteroaromatic compound can contribute to improved color stability when compared to controls of the same vehicle. Oils with a Hansen total solubility parameter (□ₜ) value within the range of 13 to 26, preferably 15 to 25, are most compatible and thus suitable for use in such cosmetic compositions. Illustrative examples of suitable oils for use in the present invention include isopropyl myristate (□ₜ =17.5), caprylic/capric triglycerides (□ₜ =18.9), isopropyl palmitate (□ₜ =17.2), Illustrative examples are sunflower seed oil, cotton oil, canola oil, grapeseed oil, soybean oil, castor oil, borage oil, olive oil, jojoba oil and mixtures thereof. Mono- and di-glycerides may also be useful. Illustrative of these categories are glyceryl monostearate and glyceryl distearate. Compatible oils are employed in the inventive composition in an amount from 15 to 30%, preferably, in an amount from 15 to 25% based on the weight of the composition.

Color stability is determined through the use of LabScan XE equipment (Hunter Associates Laboratory, Inc. Reston, Virginia). LabScan XE measures color data. Samples at different storage stages housed in vials or in measurement cells are loaded onto the LabScan XE measurement port. By following the measurement procedure specified in the associated instrument menu and computer software, colors (a*, b* and L*) are measured and calculated to result in a color change data (ΔE) value. b* is the scale of blue-yellow space based on the CIELAB color space model, wherein an increase in b* value indicates the increase in yellow color components, and the decrease of b* value indicates the increase of blue color components.

It has also been unexpectedly discovered that compositions made according to the present invention provides good stability and color stability for the composition itself, as well as anti-aging and skin-lightening benefits when applied to the skin.

Cosmetic compositions of the present invention may also comprise a cosmetically acceptable vehicle to act as a diluent, dispersant or carrier for the skin benefit agents in the composition, so as to facilitate its distribution when the composition is applied to the skin. This cosmetically acceptable vehicle may be aqueous, anhydrous or an emulsion. Oily carriers in the presence of water and an emulsifier will form emulsion systems as carriers. Preferably, the compositions are aqueous or an emulsion, especially water-in-oil or oil-in-water emulsion, preferentially oil-in-water emulsion. The cosmetic compositions ordinarily will be in but are not limited to cream or lotion form.

Suitable carriers employed in the invention include water, sea water, rosewater (e.g. *Rosa Damascena* flower Water), tea (e.g. *Camellia Sinensis* Leaf Water), *Aloe Barbadensis* (aloe vera) leaf juice, witch hazel (e.g. *Hamamelis Virginiana* extract), lavender water (e.g. *Lavendula Angustifolia* flower water), grape water (e.g. *Vitis Vinifera* fruit water and *Vitis Vinifera* juice). In the most preferred embodiment, water is the carrier. Amounts of water may preferably range from 5 to 85%, more preferably, from 15 to 75%, and most preferably, from 30 to 70%, based on the weight of the cosmetic composition and including all ranges subsumed therein. The cosmetically acceptable vehicle can, in the absence of other cosmetic adjuncts, form the balance of the composition. Besides water, suitable carrier classes include, but are not limited to, silicones, polyhydric alcohols, fatty acids, hydrocarbons, triglycerides, waxes and thickening agents.

It is within the scope of the invention to contain silicones, which may be categorized into the volatile and nonvolatile variety. Amounts may range, for example, from 0.01 to 25%, more preferably from 0.1 to 20% by weight of the cosmetic composition. In an embodiment of the invention, 0.01 to 2% by weight of the silicone is contained in the composition with no significant impact on sensory properties. The term "volatile" as used herein refers to those materials which have a measurable vapor pressure at ambient temperature. Volatile silicone oils are preferably chosen from cyclic (cyclomethicone) or linear polydimethylsiloxanes containing from 3 to 9, preferably from 4 to 5, silicon atoms.

Nonvolatile silicones useful in this composition include polyalkyl siloxanes, polyalkylaryl siloxanes and polyether siloxane copolymers. The essentially nonvolatile polyalkyl siloxanes useful herein include, for example, polydimethyl siloxanes with viscosities from about 5 x 10⁻⁶ to 0.1 m²/s at 25°C. Emulsifying and non-emulsifying silicone elastomers are also suitable for use in the cosmetic composition of this invention.

Conventional humectants, generally of the polyhydric alcohol-type materials, may be used in the present invention. Typical polyhydric alcohols include glycerol (i.e., glycerine or glycerin), propylene glycol, dipropylene glycol, polypropylene glycol, polyethylene glycol, sorbitol, hydroxypropyl sorbitol, hexylene glycol, 1,3-butylene glycol, isoprene glycol, 1,2,6-hexanetriol, ethoxylated glycerol, propoxylated glycerol and mixtures thereof. The most preferred embodiment includes glycerin, propylene glycol or a mixture thereof. The amount of humectant employed may range anywhere from 0.1 to 25%, preferably, between 0.5 and 20%, and more preferably, between 1 and 15% by weight of the cosmetic composition.

Retinoid boosters may also be useful ingredients to incorporate for use in the compositions of the present invention, such as fatty acids and derivatives thereof. As described in several U.S. Patents to Granger et al. (U.S. Pat. Nos. 5,759,556, 5,756,109, 5,747,051, 5,716,627, 5,811,110, 5,536,740, 5,747,051, 5,599,548, 5,955092, 5,885,595, 5,759,556, 5,693,330, 7,959,913, 8,226,933 and 8,409,550), the term "retinoid boosters" is used to refer collectively to compounds that have been determined to enhance the enzymatic conversion of retinyl esters and retinol to retinoic acid as part of the natural retinol metabolic process in the skin's epidermis. The boosters alone or in combination with other booster compounds help to enhance efficacy of a retinoic acid precursor. The term "fatty" refers to carbon chain lengths ranging from 10 to 30 carbon atoms. Illustrative of this category are pelargonic, lauric, myristic, palmitic, stearic, isostearic, hydroxystearic, oleic, linoleic, ricinoleic, arachidic, behenic, erucic acids and combinations thereof. Preferred derivatives of such fatty acids include fatty alcohols, such as cetyl, lauryl, myristyl, palmitic, cetearyl, stearyl and oleyl alcohols; fatty esters, such as 2-ethyl-hexyl myristate, isopropyl stearate and isostearyl palmitate, triisopropyl trilinoleate and trilauryl citrate, lauryl palmitate, myristyl lactate, oleyl eurcate and stearyl oleate, coco-caprylate/caprate (a blend of coco-caprylate and coco-caprete), propylene glycol myristyl ether acetate, diisopropyl adipate and cetyl octanoate; and fatty amides, such as cocamide monoethanolamide (CMEA), castor oil monoethanolamide, linoleoyl monoethanolamide (LAMEA), palmitamide monoethanolamide and linoleamide diethanolamide. Other illustrative but nonlimiting examples of such booster compounds include carotenoids, flavonoids, cyclic and non-cyclic fragrances, antimicotics (e.g. bifonazole, climbazole, clotrimazole, econazole, ketoconazole, miconazole), phospholipid analogues, ureas, phosphatiylethanolamine, phosphatidylcholine, sphingomyelin, natural colorants (e.g. coumarin), quinolines, isoquinolines, metyrapone and mixtures thereof. In an embodiment of the invention, fatty acids, fatty alcohols, fatty amides, climbazole, or a mixture thereof may be used as retinoid boosters. In a particularly preferred embodiment of the invention, 12-hydroxystearic acid, cetyl alcohol, cetearyl alcohol, CMEA, LAMEA, climbazole, or a mixture thereof is used as retinoid boosters. When used, from 0.01 to 5% by weight of a retinoid booster compound is present in the composition.

Waxes and wax esters are also suitable for use in the compositions of the present invention. These waxes include animal-derived waxes or those from animal byproducts (e.g. beeswax, spermaceti wax, Chinese wax, wool wax, shellac wax), plant-derived waxes (e.g. tribehenin wax, carnauba wax, candelilla wax, bayberry wax, jojoba wax, orange wax, rice bran wax, sunflower wax, castor wax, soy wax), mineral waxes (e.g. montan wax, ceresin wax, ozokerite) and synthetic derivatives of natural waxes. The amount of wax and wax esters employed may range from 0.01 to 10%, preferably from 0.1 to 8% by weight of the composition.

Hydrocarbons may be optionally included in the cosmetic composition of the present invention. Suitable hydrocarbons may include mineral oil, petrolatum and polyalpha-olefins. Examples of preferred volatile hydrocarbons include polydecanes such as isodecane and isododecane and the C₇-C₈ through C₁₂-C₁₅ isoparaffins.

Cosmetic compositions made according to the invention may optionally comprise one or more thickening agents at inclusion levels from 0.05 to 10%, preferably, from 0.1 to 5%, and more preferably, from 0.25 to 4%, by weight of the composition. Useful thickeners include polysaccharides, which comprise of starches, natural/synthetic gums and cellulosics. Suitable starches include tapioca starch, cornstarch, potato starch, aluminum starch octenylsuccinate and sodium hydroxypropyl starch phosphate. Suitable gums include xanthan, sclerotium, pectin, karaya, arabic, agar, guar, carrageenan, alginate and combinations thereof. Suitable cellulosics include hydroxypropyl cellulose, hydroxypropyl methylcellulose, ethylcellulose and sodium carboxy methylcellulose. Synthetic polymers functioning as thickening agents are also suitable for use, including polyacrylamides, Carbomers and taurate copolymers.

Emulsifiers are preferably present in the cosmetic composition of the present invention. Emulsifiers suitable for use in the present invention will have an HLB from 2.5 to 17. The HLB of the emulsifier used when oil-continuous emulsions are desired will be 2.5 to 7.5, and preferably, from 3 to 6.5, and most preferably, from 3 to 6, including all ranges subsumed therein. The HLB of the emulsifier used when water-continuous emulsions are desired will be 8 to 17, preferably, from 8.5 to 15, and most preferably, from 9 to 14, including all ranges subsumed therein.

Total concentration of the emulsifier may range from 0.1 to 20%, preferably, from 1 to 10%, and most preferably, from 1 to 8% by weight of the composition, including all ranges subsumed therein. The emulsifier may be selected from the group consisting of anionic, nonionic, cationic and amphoteric surfactants. Particularly preferred nonionic surfactants are those with a C₁₀-C₂₀ fatty alcohol or acid hydrophobe condensed with from about 2 to about 100 moles of ethylene oxide or propylene oxide per mole of hydrophobe; C₂-C₁₀ alkyl phenols condensed with from 2 to 20 moles of alkylene oxide; mono- and di-fatty acid esters of ethylene glycol; fatty acid monoglyceride; sorbitan, mono- and di- C₈-C₂₀ fatty acids; and polyoxyethylene sorbitan as well as combinations thereof. Alkyl polyglycosides and saccharide fatty amides (e.g. methyl gluconamides) are also suitable nonionic emulsifiers. Still other preferred nonionic surfactants include glyceryl stearate, glycol stearate and stearamide AMP.

Preferred anionic emulsifiers include alkyl ether sulfate and sulfonates, alkyl sulfates and sulfonates, alkylbenzene sulfonates, alkyl and dialkyl sulfosuccinates, C₈-C₂₀ acyl isethionates, C₈-C₂₀ acyl methyl isethionates, C₈-C₂₀ acyl methyl taurates, C₈-C₂₀ alkyl ether phosphates, alkyl ether carboxylates and combinations thereof.

Cationic emulsifiers that may be used include, for example, palmitamidopropyltrimonium chloride, distearyldimonium chloride, diester quaternary ammonium compounds (e.g. distearoylethyl dimonium chloride) and mixtures thereof.

Useful amphoteric emulsifiers can include cocoamidopropyl betaine, C₁₂-C₂₀ trialkyl betaines, sodium lauroamphoacetate, and sodium laurodiamphoacetate or a mixture thereof.

Fragrances, fixatives, abrasives and additional skin benefit agents may optionally be included in cosmetic compositions of the present invention. Skin benefit agents suitable for use in this invention are meant to include but not be limited to opacifiers, colorants, humectants, emollients, occlusive agents, plant extracts, optical agents, skin lightening agents, anti-inflammatory agents, anti-acne agents, antioxidants, sunscreens, photostabilizers, surfactants, wrinkle reducing agents, coloring agents, desquamation promoters, exfoliating agents, mixtures thereof or the like. Each of these substances may range from 0.05 to 5%, preferably between 0.1 and 3% by weight.

Cosmetic compositions of the present invention may include additional vitamins as the desired skin benefit agent. Illustrative vitamins are Vitamin B₂, Vitamin B₅ (panthenol), Vitamin B₆, Vitamin C, Vitamin E, Folic Acid and Biotin. Derivatives of the vitamins may also be employed. For instance, Vitamin C derivatives include ascorbyl tetraisopalmitate, magnesium ascorbyl phosphate and ascorbyl glycoside. Derivatives of Vitamin E include tocopheryl acetate, tocopheryl palmitate and tocopheryl linoleate. Total amount of additional vitamins when present in cosmetic compositions according to the present invention may range from 0.01 to 10%, preferably, from 0.05 to 7%, optimally, from 0.1 to 5% by weight of the cosmetic composition.

Other additional optional skin benefit agents suitable for use in the compositions of this invention include minerals and skin nutrients such as milk; magnesium, calcium, copper, zinc and other metallic components; alpha hydroxy acids, beta hydroxy acids, e.g. salicylic acid and derivatives thereof (such as 5-octanoyl salicylic acid, heptyloxy 4 salicylic acid, and 4-methoxy salicylic acid); kojic acid; hydroquinone and arbutin; resveratrol; saccharide isomerate; undecylenoyl phenylalanine; resorcinol derivatives (particularly 4-substituted resorcinol derivatives, including 4-ethyl resorcinol, 4-isopropyl resorcinol, 4-hexyl resorcinol, 4-cyclopentyl resorcinol, 4-cyclohexyl resorcinol and acylated forms thereof); ceramides (e.g. Ceramide 1, Ceramide 3, Ceramide 3B and Ceramide 6) and pseudoceramides; allantoin; pyroglutamic acid (PCA) salt derivatives including zinc PCA and sodium PCA; and skin benefit acids and derivatives thereof (12-hydroxystearic acid; petroselinic acid; conjugated linoleic acid; octadecanoic acid; hyaluronic acid and its salt derivatives); mixtures thereof and the like. Such skin benefit agents, when used, collectively make up from 0.001 to 12% by weight of the cosmetic composition.

A wide selection of botanical extracts may optionally be included in cosmetic compositions of this invention. The extracts may either be soluble in water or oil, carried in a solvent that is hydrophilic or hydrophobic, respectively. In the preferred embodiment, water or ethanol are the extract solvents. Illustrative examples include those extracted from green tea, yarrow, chamomile, licorice, aloe vera, citrus unshui, willow bark, alfalfa, algae, grape seed, witch hazel, sage, thyme and rosemary, as well as oils such as those derived from sea buckthorn, moringa, argan, avocado, calendula, algal and marula. Soy extracts may be used and especially when it is desirable to include retinol. Such extracts, when used, are preferably employed in collective amounts ranging from 0.001 to 12% by weight of the cosmetic composition.

It is within the scope of the invention to optionally include sunscreens and photostabilizers. The sunscreens and photostabilizers that may be used in compositions of the present invention include such materials as octylmethoxycinnamate (OMK), ethylhexyl salicylate, phenylbenzimidazole sulfonic acid (Ensulizole), ethylhexyl p-methoxycinnamate, available as Parsol MCX^{®}, Avobenzene (butyl methoxydibenzoylmethane), available as Parsol 1789^{®} and benzophenone-3, also known as oxybenzone. Inorganic sunscreen actives may be employed such as microfine titanium dioxide (preferably with a particle diameter of less than 150nm, and most preferably, less than 100nm) and zinc oxide may be used, polyethylene and various other polymers are also suitable sunscreens. Other sunscreens suitable for use include p-aminobenzoic acid (PABA), octyldimethyl-PABA, 2-ethoxyethyl p-methoxy cinnamate, benzophenone-1, benzophenone-2, benzophenone-6, benzophenone-8, benzophenone-9, benzophenone-12, homomethyl salicylate, menthyl anthranilate, benzophenone-4, triethanolamine salicylate, terephthalylidene dicamphor sulfonic acid, bisoctriazole, bisethylhexyloxyphenol methoxyphenyl triazine, bisdisulizole disodium, diometriazole trisiloxane, octyltriazone, iscotrizinol, polysilicone-15, isopentenyl-4-methoxycinnamate, mixtures thereof or the like. Also suitable for use is octocrylene. Amounts of the sunscreen or photostabilizing agents when present may generally range from 0.1 to 20%, preferably, from 0.5 to 15%, optimally, from 0.75 to 10% by weight of the cosmetic composition.

Cosmetic compositions of the present invention may also include natural and synthetic antioxidants. Illustrative but nonlimiting examples of antioxidants for use in the composition include butylated hydroxytoluene (BHT), dilauryl thiodipropionate, pentaerythrityl tetra-di-t-butyl hydroxyhydrocinnamate and polyphenols. The total weight percent of antioxidants, when used, in the present invention is, preferably, from 0.01 to 4%, more preferably, from 0.02 to 3%, and most preferably, from 0.05 to 2%.

Another optional additive suitable for use includes hemp oil with 2.5 to 25% by weight cannabigerol and/or cannabidiol at from 0.5 to 10 percent by weight. When used, such oil makes up from 0.0001 to 12% by weight of the composition, and preferably, from 0.01 to 5% by weight of the cosmetic composition, including all ranges subsumed therein.

Optionally, one or more coloring agent may also be included in cosmetic compositions of the present invention. Coloring agents include dyes or pigments of natural or synthetic origin. A dye selected for use may be organic or inorganic and water-soluble or oil-soluble. Nonlimiting examples of water-soluble dyes include, for example, D&C Yellow 8, D&C Yellow 10, D&C Orange 4, D&C Red 6, D&C Red 22, D&C Red 28, D&C Red 33, D&C Green 5 or methylene blue. Oil-soluble dyes include, for example, D&C Red 17, D&C Green 6, β-carotene, D&C Violet 2, D&C Yellow 11, D&C Orange 5 and quinoline yellow. Pigments suitable for use are specially chosen from the mineral pigments, organic pigments, lakes and mixtures thereof as known in the art. Illustrative but nonlimiting examples include mineral pigments such as titanium dioxide, ochres (yellow ochre, red ochre, brown ochre, iron hydroxide), metal oxides (zinc oxide, zirconium oxide, iron oxide, cerium oxide, chromium oxide), manganese violet, ultramarine blue, chromium hydrate and ferric blue, or metal powders (aluminum, bronze or copper powder); organic pigments such as nitroso, nitro, azo, phthalocyanine, diazine, xanthene, pyrene, quinoline, anthraquinone, triphenylmethane, fluorane, quinacridone, metal complex, isoindolinone, isoindoline, perinone, perylene, diketopyrrolopyrrole, indigo, thioindigo, dioxazine, triphenylmethane and quinophthalone compounds; and lakes (particularly those deriving from calcium, barium, aluminum and strontium salts) such as D&C Red No. 6 Barium Lake, D&C Red No. 7 Calcium Lake, D&C Red No. 27 Aluminum Lake, D&C Red No. 28 Aluminum Lake, D&C Red No. 33 Aluminum Lake, FD&C Red No. 40 Aluminum Lake, FD&C Yellow No. 5 Aluminum Lake, FD&C Yellow No. 6 Aluminum Lake, D&C Yellow No. 10 Aluminum Lake, D&C Orange No.5 Aluminum Lake and F&D Blue No. 1 Aluminum Lake. Nacres, such as natural mica coated with a metal oxide, bismuth oxychloride or a natural pigment, are also suitable to be included in cosmetic compositions of the present invention. Other coloring agents suitable for use include chalk, activated charcoal and carbon black. Cosmetic compositions comprise from 0.01 to 20%, preferably, from 0.1 to 15% and more preferably, 0.5 to 10% by weight of the coloring agent, when used, relative to the weight of the cosmetic composition.

Traditional buffers or pH modifiers are also suitable for inclusion to the cosmetic compositions of this invention. These include common additives such as sodium hydroxide, potassium hydroxide, hydrochloric acid, citric acid, triethanolamine and aminomethyl propanol. In a preferred embodiment, the pH of the cosmetic composition of the present invention is from 4 to 8, and more preferably, from 4.25 to 7.75, and most preferably, from 5 to 7.5. Viscosity of the cosmetic composition of this invention is from about 1,000 to about 200,000 cps, preferably, from about 5,000 to about 180,000 cps, and most preferably, from about 10,000 to about 150,000 cps, taken under conditions of 25°C and a shear rate of 1s⁻¹ with a strain controlled parallel plate rheometer made commercially available from suppliers like T.A. Instruments under the Discovery name. Alternatively, viscosity can also be measured using a Brookfield Viscometer (speed at 20 rpm, spindle 5, helipath off, for one (1) minute at 25°C). The cosmetic composition of the invention can be formulated as a lotion having a viscosity from 4,000 to 10,000 mPas, a fluid cream having a viscosity from 10,000 to 20,000 mPas or a cream having a viscosity from 20,000 to 200,000 mPas or above.

Preservatives can be incorporated into the compositions of this invention as desired to protect against the growth of potentially harmful microorganisms. Cosmetic chemists are familiar with appropriate preservatives and routinely choose them to satisfy preservative tests and product stability tests. Preservative systems should be selected having regard for the use of the composition and possible incompatibilities between the preservatives and other ingredients in the formulation. Exemplary examples of preservatives for compositions of this invention include, without limitations, iodopropynyl butyl carbamate (IPBC), phenoxyethanol, ethylhexylglycerine, 1,2-octanediol, methyl paraben, propyl paraben, imidazolidinyl urea, sodium dehydroacetate, propanediol, alkyl esters of para-hydroxybenzoic acid, sodium benzoate, benzoic acid, hydroxyacetophenone, DMDM hydantoin derivatives, propionate salts, and a variety of quaternary ammonium compounds. Preservatives are preferably employed in amounts ranging from 0.01 to 2% by weight of the composition, including all ranges subsumed therein.

When making the cosmetic composition of the present invention, the desired ingredients are mixed via conventional methods. Cosmetic compositions are made by mixing the ingredients, in no particular order, under conditions of moderate sheer, about 22°C to about 85°C, and atmospheric pressure.

The cosmetic composition of this invention is a composition suitable for topical application to human skin, including leave-on and wash-off products. Preferably, the term encompasses a fluid liquid, and particularly a moisturizer rather than a make-up product. Most preferred are leave-on compositions. The term "leave-on" as used with reference to compositions herein means a composition that is applied to or rubbed on the skin and left thereon.

In another embodiment, the present invention is directed to the use of an oil having a Hansen total solubility parameter value in the range of 13 to 26 to stabilize a composition comprising a retinoic acid precursor and a functionalized heteroaromatic compound.

Many types of packaging can be used to store and deliver the composition of the present invention.

The selection of packaging is dependent upon the personal care end-use and the viscosity of the composition itself. As an example, leave-on lotions and creams for skin typically employ plastic containers with an opening at a dispense end covered by an appropriate closure. Conventional closures include flip-top hinged lids, screw-caps and non-aerosol pumps. As another example, appropriate packaging to be used for antiperspirants, deodorants and depilatories include a container with a roller-ball applicator on a dispensing end if the composition is fluid and of a thinner viscosity. If the composition is in a stick format, a container with a propel-repel mechanism wherein the stick is fixed on a platform towards a dispensing orifice is appropriate. If the composition is in an aerosol format, then metallic cans pressurized by a propellant and having a spray nozzle is appropriate. In general, patches, bottles, tubes, roller-ball applicators, squeeze containers or lidded jars are preferred. When plastic is desired, it is preferably post-consumer resin.

The invention will now be described below in the context of specific examples to facilitate a greater understanding of the present invention.

### Examples

All samples were made by mixing the mentioned ingredients under conditions of moderate sheer, about 22°C to about 85°C, and atmospheric pressure. All □ₜ data are collected from the CRC Handbook of Solubility Parameters and Other Cohesion Parameters, Second Edition (by Allan F.M Barton).

### Example 1: Formulation with 3% niacinamide and 0.3% retinyl propionate

| Ingredient | wt. % |
|---|---|
| Water | Balance |
| Glycerin | 7.00 |
| Chelator | 0.10 |
| Thickener | 2.00 |
| Niacinamide | 3.00 |
| Preservatives | 0.70 |
| Cetyl alcohol | 0.50 |
| Emulsifiers | 2.50 |
| Antioxidants | 0.10 |
| Oil | 17 |
| Retinyl Propionate | 0.30 |

An emulsion cosmetic composition was made by combining the ingredients above according to the present invention.

### Example 2: Additional sample formulations

| Ingredient | wt. % | | | | |
|---|---|---|---|---|---|
| | Sample 1 | Sample 2 | Sample 3 | Sample 4 | Sample 5 |
| Water | Balance | Balance | Balance | Balance | Balance |
| Glycerin | 3.00 | 3.00 | 3.00 | 5.00 | - |
| Propylene Glycol | - | - | - | - | 7.00 |
| Chelator | 0.10 | 0.10 | 0.10 | 0.10 | 0.10 |
| Thickener | 2.00 | 2.00 | 2.00 | 2.50 | 2.50 |
| Niacinamide | 1.00 | 1.50 | 1.50 | 2.00 | - |
| Picolinamide | - | - | - | 1.00 | 3.00 |
| Preservatives | 0.70 | 0.70 | 0.70 | 0.70 | 0.70 |
| Cetearyl alcohol | - | - | - | - | 0.50 |
| 12-hydroxystearic acid | - | - | - | 0.50 | - |
| Cetyl alcohol | - | 0.25 | 0.25 | - | - |
| Emulsifiers | 2.50 | 2.50 | 2.50 | 2.50 | 2.50 |
| Antioxidants | 0.10 | 0.10 | 0.10 | 0.10 | 0.10 |
| Oil | 15 | 15 | 15 | 19 | 19 |
| Retinyl Propionate | 0.10 | - | - | 0.20 | - |
| Retinol | - | 0.10 | - | 0.10 | 0.30 |
| Retinyl Palmitate | - | - | 0.10 | - | - |

Additional cosmetic compositions may be made by combining the ingredients above, which are consistent with the described invention.

### Example 3: Color stability with varying amounts of oil

| Oil (wt.%) | ΔE | | |
|---|---|---|---|
| | 4 weeks | 8 weeks | 12 weeks |
| 0 | 57.69 | 68.34 | 66.28 |
| 5 | 11.91 | 20.56 | 25.45 |
| 10 | 7.19 | 13.75 | 17.36 |
| 17 | 3.04 | 5.98 | 8.06 |
| 20 | 3.15 | 6.18 | 9.18 |

As can be seen from the data, oil used consistent with the invention unexpectedly stabilizes a formulation with RP at 0.3% and niacinamide at 3%. The formulations with more than 10% oil yields lower ΔE values over time than that with 5% oil or no oil, with surprisingly superior color stability occurring with inclusion levels around 17 to 20%.

### Example 4: Color and chemical stability of retinyl propionate (RP) with varying oils and levels of niacinamide

| Samp le | Oil (wt.%) | | | | Vitam in B₃ (wt.% ) | ΔE, 4 weeks @45° C | ΔE, 8 week s @45° C | ΔE, 12 weeks @45° C | RP Remain ing, % @ 4 weeks | RP Remain ing, % @ 12 weeks |
|---|---|---|---|---|---|---|---|---|---|---|
| | Dimethic one 5cSt (□ₜ = 6.0) | Isopro pyl Myrist ate (□ₜ =17.5) | Caprylic/ Capric Triglycer ide (□ₜ =18.9) | Benzyl Alcoho I (□ₜ =24.7) | | | | | | |
| 1. | 17 | - | - | - | 0.1 | 5.22 | 8.25 | 10.08 | 89.99 | 81.98 |
| 2) | 17 | - | - | - | 1.0 | 10.44 | 16.68 | 20.00 | 93.52 | 81.8 |
| 3) | 17 | - | - | - | 2.0 | 13.32 | 20.53 | 25.53 | 92.14 | 79.58 |
| 4) | 17 | - | - | - | 3.0 | 15.84 | 24.14 | 29.42 | 93.12 | 80.34 |
| 5) | - | 17 | - | - | 0.1 | 0.49 | 2.11 | 3.06 | 96.93 | 89.56 |
| 6) | - | 17 | - | - | 1.0 | 1.31 | 3.49 | 5.24 | 95.28 | 87.74 |
| 7) | - | 17 | - | - | 2.0 | 2.40 | 5.22 | 7.16 | 95.13 | 86.05 |
| 8) | - | 17 | - | - | 3.0 | 3.04 | 5.98 | 8.06 | 94.12 | 86.19 |
| 9) | - | - | 17 | - | 3.0 | 3.62 | 7.25 | 10.02 | - | - |
| 10) | - | - | - | 17 | 3.0 | 66.17 | 64.48 | 63.38 | - | - |
| 11) | 8.5 | 8.5 | - | - | 1.0 | 2.76 | 6.83 | 8.80 | 94.64 | 85.5 |
| 12) 1% Cetyl Alcohol | - | 17 | - | - | 1.0 | 1.91 | 4.15 | 5.84 | 96.76 | 90.51 |

All samples in Example 4 were made to include a total oil content (by weight percent) of 17% in the composition.

From the data, it is noted that increasing inclusion levels of niacinamide resulted in greater degradation of color over time and thereby higher ΔE values. The different identities of oily solvents, which corresponds to differing Hansen solubility parameter values, employed in this formulation can result in significant differences in color and/or chemical stability wherein it is again seen that oils with □ₜ values consistent with the invention yield lower ΔE values (i.e. superior color stability) over time. For instance, isopropyl myristate and caprylic/capric triglyceride, both which possess □ₜ values within scope of the invention, demonstrate greater color stability compared to dimethicone and benzyl alcohol,

Columns designating "RP remaining" refer to the chemical efficacy of retinyl proprionate over time as a direct measurement of the level of retinyl proprionate after storage and degradation. This measurement is included to ensure that there is still enough of the bioactive to provide the biofunctionality desired. It is clear that the amount of RP remaining at the end of 12 weeks does not fluctuate tremendously from sample to sample regardless of the type of oil used or amount of niacinamide in the composition.

Sample 12 additionally contains a retinoid booster, cetyl alcohol, at 1% on top of the formulation used in Sample 6. Results show that though the measurable chemical activity of retinyl proprionate of Sample 12 is better that of all other samples, Sample 12 is comparable to, but unexpectedly less color stable than Sample 6.

### Example 5: Color stability comparisons with competitor products containing RP

| Product | b* value @ 0 Weeks | b* value @ 4 Weeks |
|---|---|---|
| Current invention composition with 0.1% RP | 2.12 | 7.17 |
| Olay^{®} Regenerist Retinol 24 Night Moisturizer | 4.22 | 10.0 |

Comparison of a composition made according to the present invention with a competitor product benchmark containing retinyl proprionate as the retinoic acid precursor shows significantly lower b* values, which indicate less yellowing and thus less color degradation, for compositions made in scope with the present invention. The competitor product selected in this Example also further contained niacinamide and retinol, but not a compatible oil.

### Example 6: Color stability comparisons of competitor product containing retinol

| Product | b* value @ 0 Weeks | b* value @ 4 Weeks |
|---|---|---|
| Current invention composition with 0% RP + 0.1% retinol | 3.82 | 9.76 |
| RoC^{®} RETINOL CORREXION^{®} Max Daily Hydration Crème | 10.84 | 15.38 |

Comparison of a composition made according to the present invention with a competitor product benchmark containing retinol as the retinoic acid precursor shows significantly lower b* values at 0 and 4 weeks, demonstrating less yellowing and thereby less color degradation, for compositions made in scope with the present invention. The competitor product selected in this Example did not contain niacinamide or any other functionalized heteroaromatic compound, nor a compatible oil.

### Example 7: Color stability of competitor products containing retinyl palmitate

| Product | b* value @ 0 Weeks | b* value @ 4 Weeks |
|---|---|---|
| Current invention composition with 0% RP + 0.1% retinyl palmitate | 2.04 | 2.24 |
| L'Oréal Paris^{®} RevitaLift^{®} Anti-Wrinkle + Firming Day Cream SPF 25 | 12.49 | 15.05 |
| L'Oréal Paris^{®} RevitaLift^{®} Anti-Wrinkle + Firming Night Moisturizer | 8.270 | 12.52 |

Comparison of a composition made according to the present invention with a competitor product benchmark containing retinyl palmitate as the retinoic acid precursor shows significantly lower b* values at 0 and 4 week time points, which indicate less yellowing and thus less color degradation, for compositions made in scope with the present invention. The competitor products selected in this Example did not contain niacinamide or any other functionalized heteroaromatic compound, nor a compatible oil.

## Claims

1. A cosmetic composition comprising:
(a) a retinoic acid precursor wherein the retinoic acid precursor comprises retinyl esters, retinol, retinal and mixtures thereof;
(b) a functionalized heteroaromatic compound; and
(c) an oil where the oil has a Hansen total solubility parameter from 13 to 26; wherein the functionalized heteroaromatic compound comprises of a carboxylic acid functionalized heteroaromatic compound; wherein the carboxylic acid functionalized heteroaromatic compound comprises of nicotinic acid, picolinic acid, nicotinate, niacinamide, picolinamide or a mixture thereof; wherein the oil makes up from 15 to 30% by weight of the composition, and wherein the oil is isopropyl myristate, isopropyl palmitate, caprylic/capric triglycerides, or a mixture thereof.

2. The cosmetic composition according to Claim 1, wherein the composition comprises 0.001 to 10% by weight of the retinoic acid precursor or mixture thereof.

3. The cosmetic composition according to any of the preceding claims, wherein the composition comprises 0.01 to 10% by weight of the functionalized heteroaromatic compound.

4. The cosmetic composition according to any of the preceding claims, wherein the composition comprises 15 to 25% by weight oil.

5. The cosmetic composition according to any of the preceding claims, wherein the composition further comprises at least one of a sunscreen, photostabilizer, skin-lightening agent, wrinkle-reducing agent, coloring agent or a mixture thereof.

6. The cosmetic composition according to any of the preceding claims, wherein the composition further comprises a retinoid booster wherein the retinoid booster comprises 12-hydroxystearic acid, cetyl alcohol, cetearyl alcohol, cocamide monoethanolamide, linoleoyl monoethanolamide, climbazole, or a mixture thereof.

7. The cosmetic composition according to Claim 1, wherein the composition further comprises 0.01 to 25% by weight silicone, preferably 0.01 to 2% by weight silicone.

## Patentansprüche

1. Kosmetische Zusammensetzung, umfassend:
(a) einen Retinsäure-Vorläufer, wobei der Retinsäure-Vorläufer Retinylester, Retinol, Retinal und Mischungen davon umfasst;
(b) eine funktionalisierte heteroaromatische Verbindung; und
(c) ein Öl, wobei das Öl einen Hansen-Gesamtlöslichkeitsparameter von 13 bis 26 aufweist;
wobei die funktionalisierte heteroaromatische Verbindung eine mit Carbonsäure funktionalisierte heteroaromatische Verbindung umfasst;
wobei die mit Carbonsäure funktionalisierte heteroaromatische Verbindung Nikotinsäure, Picolinsäure, Nicotinat, Niacinamid, Picolinamid oder einer Mischung davon umfasst;
wobei das Öl 15 bis 30 Gew.-% der Zusammensetzung ausmacht und wobei das Öl Isopropylmyristat, Isopropylpalmitat, Caprylic/Capric-Triglyceride oder eine Mischung davon ist.

2. Kosmetische Zusammensetzung nach Anspruch 1, wobei die Zusammensetzung 0,001 bis 10 Gew.-% des Retinsäure-Vorläufers oder einer Mischung davon umfasst.

3. Kosmetische Zusammensetzung nach einem der vorhergehenden Ansprüche, wobei die Zusammensetzung 0,01 bis 10 Gew.-% der funktionalisierten heteroaromatischen Verbindung umfasst.

4. Kosmetische Zusammensetzung nach einem der vorhergehenden Ansprüche, wobei die Zusammensetzung 15 bis 25 Gew.-% Öl umfasst.

5. Kosmetische Zusammensetzung nach einem der vorhergehenden Ansprüche, wobei die Zusammensetzung ferner mindestens ein Sonnenschutzmittel, einen Photostabilisator, ein Mittel zur Hautaufhellung, ein Mittel zur Faltenreduktion, ein Farbmittel oder eine Mischung davon umfasst.

6. Kosmetische Zusammensetzung nach einem der vorhergehenden Ansprüche, wobei die Zusammensetzung ferner einen Retinoid-Booster umfasst, wobei der Retinoid-Booster 12-Hydroxystearinsäure, Cetylalkohol, Cetearylalkohol, Cocamidmonoethanolamid, Linoleoyl-monoethanolamid, Climbazol oder eine Mischung davon umfasst.

7. Kosmetische Zusammensetzung nach Anspruch 1, wobei die Zusammensetzung ferner 0,01 bis 25 Gew.-% Silikon enthält, vorzugsweise 0,01 bis 2 Gew.-% Silikon.

## Revendications

1. Composition cosmétique comprenant :
(a) un précurseur d'acide rétinoïque, lequel précurseur d'acide rétinoïque comprend des esters rétinyliques, du rétinol, du rétinal et leurs mélanges ;
(b) un composé hétéroaromatique fonctionnalisé ; et
(c) une huile, laquelle huile a un paramètre de solubilité totale de Hansen de 13 à 26 ;
dans laquelle le composé hétéroaromatique fonctionnalisé comprend un composé hétéroaromatique fonctionnalisé par un acide carboxylique ; dans laquelle le composé hétéroaromatique fonctionnalisé par un acide carboxylique comprend l'acide nicotinique, l'acide picolinique, un nicotinate, le niacinamide, le picolinamide ou un mélange de ceux-ci ; dans laquelle l'huile représente 15 à 30 % en poids de la composition, et dans laquelle l'huile est le myristate d'isopropyle, le palmitate d'isopropyle, les triglycérides capryliques/capriques, ou un mélange de ceux-ci.

2. Composition cosmétique selon la revendication 1, laquelle composition comprend 0,001 à 10 % en poids du précurseur d'acide rétinoïque ou d'un mélange de tels précurseurs.

3. Composition cosmétique selon l'une quelconque des revendications précédentes, laquelle composition comprend 0,01 à 10 % en poids du composé hétéroaromatique fonctionnalisé.

4. Composition cosmétique selon l'une quelconque des revendications précédentes, laquelle composition comprend 15 à 25 % en poids d'huile.

5. Composition cosmétique selon l'une quelconque des revendications précédentes, laquelle composition comprend en outre au moins l'un parmi un écran solaire, un photostabilisant, un agent éclaircissant la peau, un agent réduisant les rides, un agent colorant, ou un mélange de ceux-ci.

6. Composition cosmétique selon l'une quelconque des revendications précédentes, laquelle composition comprend en outre un potentialisateur de rétinoïde, dans laquelle le potentialisateur de rétinoïde comprend l'acide 12-hydroxystéarique, l'alcool cétylique, l'alcool cétéarylique, le coco-amidomonoéthanolamide, le linoléoyl-monoéthanolamide, le climbazole, ou un mélange de ceux-ci.

7. Composition cosmétique selon la revendication 1, laquelle composition comprend en outre 0,01 à 25 % en poids de silicone, de préférence 0,01 à 2 % en poids de silicone.
